# FASCICULE DE BREVET EUROPEEN

(11) **EP 3 700 629 B1**
(45) Date de publication et mention de la délivrance du brevet: **17.01.2024**
(21) Numéro de dépôt: 18789134.6
(22) Date de dépôt: 18.10.2018
(51) Int. Cl.: A61N 7/02, A61N 7/00, A61B 90/00, A61B 17/22

(54) **APPAREIL DE TRAITEMENT DE LA THROMBOSE VASCULAIRE PAR ULTRASONS**
VORRICHTUNG ZUR BEHANDLUNG VON GEFÄSSTHROMBOSE MITTELS ULTRASCHALL
APPARATUS FOR TREATING VASCULAR THROMBOSIS BY ULTRASOUNDS

(30) Priorité: 23.10.2017 FR 1759995
(43) Date de publication de la demande: 02.09.2020
(73) Titulaire: Cardiawave SA, 75017 Paris (FR)
(72) Inventeur: GOUDOT, Guillaume, 75015 Paris (FR); PERNOT, Mathieu, 75004 Paris (FR); TANTER, Mickael, 92220 Bagneux (FR); VION, Michael, 41260 La Chaussee Saint Victor (FR)
(74) Mandataire: Atout PI Laplace
(86) Numéro de dépôt international: PCT/EP2018/078510
(87) Numéro de publication internationale: WO 2019/081329

(56) Documents cités:
- EP-A1- 0 614 651
- US-A- 5 520 188
- US-A- 5 827 204
- US-A1- 2004 138 563
- US-A1- 2012 022 552
- US-A1- 2012 029 353
- US-B2- 8 277 398

## Description

L'invention porte sur un appareil de traitement de la thrombose vasculaire par ultrasons, et plus particulièrement par histotripsie.

L'occlusion d'un vaisseau, veine ou artère, par un processus de thrombose aiguë (formation d'un caillot, ou thrombus), est un mécanisme fréquent et grave, responsable de la plupart des causes de mortalité dans le monde. Dans sa partie artérielle, la thrombose est à l'origine de la majorité des accidents vasculaires cérébraux et infarctus du myocarde. Lorsqu'elle survient dans le réseau veineux, la thrombose provoque des douleurs, des oedèmes, ainsi qu'un risque de détachement du caillot, responsable d'une embolie pulmonaire. La persistance de l'occlusion de la veine par un thrombus peut être, à terme, responsable d'une insuffisance veineuse chronique, se manifestant par des douleurs, un oedème, des ulcères, responsable d'une altération importante de la qualité de vie.

Le principe de traitement de la thrombose, artérielle ou veineuse, repose sur l'anticoagulation systémique permettant d'éviter l'extension de la thrombose et les complications embolique. Lors d'une thrombose veineuse profonde, ce traitement est cependant sans effet sur l'insuffisance veineuse chronique, car il ne permet le plus souvent pas la recanalisation de la veine thrombosée. L'utilisation d'un traitement thrombolithique peut permettre de recanaliser la veine occluse mais l'administration systémique est dangereuse du fait de l'induction d'hémorragies. L'utilisation de ce traitement n'est donc pas recommandée en cas de thrombose veineuse sans embolie pulmonaire grave. L'utilisation de la thrombolyse in situ, par administration locale par cathéter, permet de limiter les effets systémiques. Ces techniques sont invasives et nécessitent le plus souvent la pose de matériel étranger (stent le plus souvent). En conséquence il existe des risques locaux (saignement, infection) et d'occlusion à distance du fait de la persistance de matériel endovasculaire. Une approche thérapeutique alternative est constituée par l'utilisation d'ondes ultrasonores, seules ou en association avec des médicaments. En particulier, il est connu d'appliquer la technique connue sous le nom d'histotripsie pour fractionner des caillots de manière à rétablir la circulation dans un vaisseau sanguin obstrué ; on parle alors de « thrombotripsie ». L'histotripsie consiste à utiliser des impulsions intenses et focalisées d'ultrasons pour induire une cavitation localisée qui fractionne mécaniquement un tissu mou cible.

Le document US 5,827,204 décrit un appareil de thérapie par ultrasons pouvant notamment être utilisé pour une traitement de thrombotripsie. L'appareil comprend un transducteur de thérapie associé à un transducteur d'imagerie, les deux transducteurs étant reliés à un actionneur permettant leur déplacement dans une direction axiale.

La demande internationale WO 2009/094554 et les articles :
- Xi Zhang et al. « Histotripsy thrombolysis on retracted clots », Ultrasound in Med. & Biol. Volume 42, Issue 8, août 2016, pages 1903-1918;
- Xi Zhang et al. « Non-invasive thrombolysis using histotripsy in a porcine deep vein thrombosis model », Ultrasound in Med. & Biol. 43, Issue 7, juillet 2017 ; et
divulguent des techniques de thrombotripsie qui ont été validée par des études *in vitro* et - dans l'article de 2017 - *in vivo* sur des cochons.

Dans ces cas les ultrasons de thérapie, à une fréquence de 1 MHz, sont appliqués au moyen d'un transducteur « de thérapie » multiéléments à longueur focale fixe, au centre duquel est agencé un transducteur d'imagerie par ultrasons (échographie). Dans les études *in vitro* tant les transducteurs que le capillaire modélisant la veine à traiter sont immergés dans un liquide de couplage acoustique. Dans les études *in vivo* les transducteurs sont immergés dans un liquide de couplage acoustique confiné par un bol sans fond et une feuille en matière plastique, en contact avec la peau de l'animal. Le transducteur d'imagerie permet de localiser la veine à traiter et la tache focale du transducteur de thérapie, cette dernière étant rendue visible par le nuage de bulles de cavitation qu'elle engendre. Puis l'ensemble transducteur de thérapie - transducteur d'imagerie est déplacé mécaniquement jusqu'à ce que la tache focale coïncide avec la veine.

L'article de K. B. Bader et al. « Efficacy of histotripsy combined with rt-PA in vitro », Phys. Med. Biol., 21 juillet 2016 étudie les conditions opératoires optimales pour une méthode de traitement de la thrombose veineuse associant histotripsie et administration de médicaments thrombolithiques. Seules des études *in vitro* sont présentées, utilisant un transducteur de thérapie de type multi-annulaire, un transducteur d'imagerie (dissocié du précédent) et un capillaire modélisant une veine immergés dans un liquide de couplage.

Le couplage acoustique par immersion des transducteurs dans un liquide est peu pratique. En outre, le capteur d'imagerie doit nécessairement être maintenu à distance de la peau du patient, ce qui dégrade la qualité des images acquises. De plus, cette distance n'est pas constante - et la qualité des images est donc variable. Cela nuit à la précision, et donc à l'efficacité, du traitement, et augmente le risque d'endommagement de la paroi du vaisseau sanguin soumis au traitement par les ultrasons focalisés.

L'invention vise à surmonter les inconvénients précités de l'art antérieur. Plus particulièrement elle vise à procurer un appareil permettant une thrombotripsie assistée par imagerie plus simple à mettre en oeuvre, permettant une meilleure précision du traitement et réduisant notamment le risque d'endommagement de la paroi du vaisseau sanguin.

Conformément à l'invention ce but est atteint en permettant, par des moyens mécaniques et/ou électroniques, un déplacement de la tache focale du transducteur ultrasonore de thérapie par rapport au transducteur ultrasonore d'imagerie suivant l'axe d'émission. Cela permet de maintenir le transducteur d'imagerie en contact avec la surface du corps humain ou animal à traiter - ou plus généralement à une distance sensiblement constante de ce dernier - pendant le balayage de la région à traiter et, par conséquent d'obtenir des images de meilleure qualité. A son tour, la meilleure qualité des images permet de localiser plus précisément la paroi du vaisseau sanguin à traiter, et donc d'éviter son endommagement accidentel.

Selon un mode de réalisation avantageux de l'invention ce but peut également être atteint grâce à l'utilisation d'un transducteur ultrasonore de thérapie opérant à une fréquence supérieure ou égale à 2 MHz, au lieu d'une fréquence de l'ordre de 1 MHz comme dans l'art antérieur. Cela permet d'obtenir une tache focale plus petite et plus stable, permettant de tirer pleinement parti de la meilleure localisation de la paroi du vaisseau sanguin afin d'éviter son endommagement.

Un objet de l'invention est donc un appareil de traitement de la thrombose vasculaire par ultrasons, comprenant :
- un transducteur ultrasonore de thérapie, adapté pour générer des ondes ultrasonores focalisées se propageant selon un axe d'émission ;
- un transducteur ultrasonore d'imagerie associé au transducteur ultrasonore de thérapie, adapté pour acquérir des images bi- ou tridimensionnelles d'une région à traiter d'un corps humain ou animal, la région à traiter incluant une tache focale du transducteur ultrasonore de thérapie ;
- un moyen de déplacement de la tache focale du transducteur ultrasonore de thérapie suivant l'axe d'émission par rapport au transducteur ultrasonore d'imagerie ;
- un système mécanique motorisé adapté pour déplacer le transducteur ultrasonore de thérapie et le transducteur ultrasonore d'imagerie en translation suivant au moins un premier axe de déplacement parallèle audit axe d'émission, et en translation ou en rotation selon ou autour un deuxième axe de déplacement non parallèle au premier ; et
- un système électronique de contrôle configuré pour :
   - piloter le système mécanique motorisé de manière à effectuer un balayage de la région à traiter tout en maintenant constante, avec une tolérance prédéfinie, une distance entre le transducteur ultrasonore d'imagerie et une surface du corps humain ou animal ; et
   - piloter le moyen de déplacement la tache focale du transducteur ultrasonore de thérapie de manière à contrôler la position de la tâche focale selon l'axe d'émission au cours dudit balayage.

Selon des modes de réalisation particuliers de l'invention :
- L'appareil peut comprendre également un capteur de force adapté pour générer un signal indicatif d'une force s'exerçant sur le transducteur ultrasonore d'imagerie suivant une direction parallèle audit axe d'émission, le système électronique de contrôle étant configuré pour acquérir ce signal et l'utiliser pour piloter le système mécanique motorisé de manière à maintenir ledit transducteur ultrasonore d'imagerie en contact avec ladite surface du corps humain ou animal au cours du balayage.
- Le système électronique de contrôle peut également être configuré pour appliquer ledit transducteur ultrasonore d'imagerie sur ladite surface du corps humain ou animal au cours du balayage avec une force constante.
- Le système électronique de contrôle peut également être configuré pour analyser des images acquises par le transducteur ultrasonore de thérapie de manière à détecter la surface dudit corps humain ou animal afin de piloter le système mécanique motorisé lors dudit balayage.
- Le système électronique de contrôle peut également être configuré pour piloter le moyen de déplacement de la tache focale du transducteur ultrasonore de thérapie de telle sorte que ladite tache focale suive un chemin prédéfini à l'intérieur dudit corps humain ou animal au cours du balayage.
- Le système électronique de contrôle peut également être configuré pour :
   a) piloter le système mécanique motorisé de manière à effectuer un premier balayage de la région à traiter ;
   b) au cours de ce premier balayage, acquérir une pluralité d'images de ladite région tout en maintenant le transducteur ultrasonore de thérapie inactif ;
   c) piloter le système mécanique motorisé de manière à effectuer un second balayage de la région à traiter ;
   d) au cours de ce deuxième balayage, activer le transducteur ultrasonore de thérapie et piloter le moyen de déplacement sa tache focale de telle sorte que ladite tache focale suive un chemin prédéfini à l'intérieur dudit corps humain, identifié à partir des images acquises au cours du premier balayage.
- Plus particulièrement, le système électronique de contrôle peut également être configuré pour:
   b1) analyser les images acquises au cours du premier balayage pour identifier un vaisseau sanguin dans la région à traiter dudit corps humain ou animal ; et
   b2) déterminer ledit chemin prédéfini à l'intérieur dudit corps humain de telle sorte qu'il correspond audit vaisseau sanguin.
- En outre, le système électronique de contrôle peut également être configuré pour piloter le système mécanique motorisé de telle manière que, au cours du deuxième balayage, ledit vaisseau sanguin se trouve dans une partie centrale d'un champ de vision du transducteur ultrasonore d'imagerie.
- Le système électronique de contrôle peut également être configuré pour acquérir une pluralité d'images de la région à traiter au cours du deuxième balayage et pour utiliser ces images pour piloter le système mécanique motorisé.
- Le transducteur ultrasonore de thérapie peut être un transducteur multiélément et le moyen de déplacement de sa tache focale comprend un formateur électronique de faisceau configuré pour piloter les éléments dudit transducteur avec des retards variables afin d'émettre les ondes ultrasonores focalisées avec une longueur focale réglable. Plus particulièrement, il peut s'agir d'un transducteur concentrique multi-annulaire.
- Le moyen de déplacement de la tache focale du transducteur ultrasonore de thérapie peut comprendre un système mécanique permettant un déplacement relatif, selon ledit axe d'émission, du transducteur ultrasonore de thérapie et du transducteur ultrasonore d'imagerie.
- Le transducteur ultrasonore d'imagerie peut dépasser, dans une direction parallèle audit axe d'émission, du transducteur ultrasonore de thérapie.
- Le transducteur ultrasonore d'imagerie peut être agencé au centre du transducteur ultrasonore de thérapie.
- L'appareil peut comprendre également un dispositif d'interface acoustique adapté pour coupler les ondes ultrasonores focalisées générées par le transducteur ultrasonore de thérapie à ladite surface du corps humain ou animal.
- Ledit système mécanique motorisé peut présenter trois degrés de liberté en translation et trois degrés de liberté en rotation.
- Ledit transducteur ultrasonore de thérapie peut être adapté pour générer des ondes ultrasonores focalisées à une fréquence supérieure ou égale à 2 MHz.
- Le système électronique de contrôle et le transducteur ultrasonore d'imagerie peuvent également être configurés pour surveiller la recanalisation d'un vaisseau sanguin traité par imagerie Doppler.

D'autres caractéristiques, détails et avantages de l'invention ressortiront à la lecture de la description faite en référence aux dessins annexés donnés à titre d'exemple et qui représentent, respectivement :
- la figure 1, un schéma d'un appareil selon un mode de réalisation de l'invention ; et
- la figure 2, un ordinogramme d'une méthode de traitement utilisant un tel appareil.

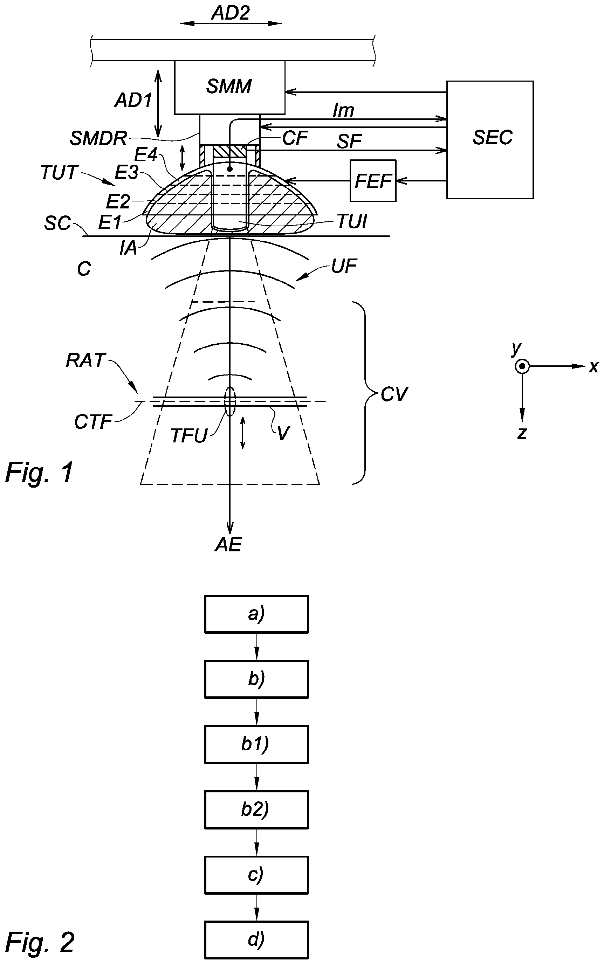

Dans le dispositif de la figure 1, le transducteur ultrasonore de thérapie TUT est du type « multiéléments annulaire », c'est-à-dire qu'il est constitué d'un ensemble de transducteurs ultrasonores, ou éléments, annulaires (références E1, E2, E3, E4) de rayons décroissants, agencés de manière concentriques de manière à former une calotte sphérique, ou plus généralement concave (par exemple en forme de paraboloïde). Un tel transducteur est décrit par exemple dans le document US 5,520,188.

Bien entendu, le nombre de transducteurs n'est pas nécessairement égal à 4 ; de préférence, ce nombre sera compris entre 4 et 20 pour éviter une complexité excessive.

Les éléments annulaires sont de préférence circulaires ; plus précisément ils peuvent présenter une forme tronconique à base circulaire, mais d'autres formes sont envisageables. Ils peuvent être continus ou être formés de segments discrets.

Les ondes ultrasonores UF générées par les éléments E1 - E4 du transducteur de thérapie TUT sont focalisées en une tache focale TFU ayant une forme généralement ellipsoïdale. On désigne par « z » l'axe principal d'émission des ultrasons. Dans un souci de simplicité, bien que cela ne soit pas essentiel, on considérera que cet axe est perpendiculaire à la surface SC (c'est-à-dire de la peau) du corps humain ou animal C à traiter. Le couplage des ultrasons au corps est assuré par une interface acoustique IA, formé par exemple par une poche en matière plastique souple remplie de gel ou d'eau dégazée. Cette poche présente une face convexe en contact avec les éléments du transducteur et une face sensiblement plane destinée à rentrer en contact avec la surface SC. En utilisation cette face plane peut être à son tour induite d'un gel de couplage acoustique.

La forme concave du transducteur assure la focalisation des ultrasons UF à une distance focale « naturelle », ou « géométrique » le long de l'axe z. Une focalisation électronique, obtenue en introduisant des déphasages entre les signaux de pilotage des éléments, permet de modifier finement cette longueur focale. Dans le mode de réalisation de la figure 1, ces signaux de pilotage sont générés par un système électronique de contrôle SEC et les décalages sont introduits par un circuit formateur de faisceau FEF, piloté par le système SEC.

Avantageusement, le transducteur de thérapie est configuré pour opérer à une fréquence supérieure ou égale à 2 MHz, par exemple égale à 2,25 MHz, alors que dans l'art antérieur précité la fréquence des ondes ultrasonores de thérapie est plutôt de l'ordre de 1 MHz. En effet, pour une géométrie du transducteur et une distance focale données, la largeur (perpendiculairement à la direction de propagation) et la longueur (parallèlement à cette longueur) de la tache focale sont inversement proportionnelles à la fréquence. Ce résultat théorique est confirmé par des simulations numériques. Ainsi, en opérant à 2 MHz plutôt qu'à 1 MHz il est possible de diviser le volume de la tache focale d'un facteur 8. Cela permet de mieux cibler le caillot à désagréger et de minimiser les risques d'endommagement des parois du vaisseau sanguin.

Des essais *in vitro* ont confirmé l'efficacité des ultrasons à 2,25 MHz dans un traitement de thrombotripsie. Lors de ces essais, du sang humain a été placé dans des tubes en silicone de 6 mm de diamètre interne, modélisant des veines fémorales humaines, maintenus en position verticale. La coagulation du sang a été induite en ajoutant 20 nM de chlorure de calcium, conduisant à la formation de caillots de 2,5 cm de longueur. Ensuite les tubes ont été placés en position horizontale et chargés de solution saline (0,9% NaCl) à une pression de 30 cm H₂O. Seuls les tubes contenant un caillot obstructif ont été retenus et soumis à un traitement de thrombotripsie. Ce traitement a été appliqué au moyen de deux transducteurs ultrasonores opérant à 2,25 MHz, présentant une longueur focale de 38 mm et un diamètre également de 38 mm. Les tubes, les transducteurs de thrombotripsie et un transducteur d'imagerie ont été placés dans un bain d'eau dégazée. Trois protocoles de thérapie ont été testés : 3 passages à une vitesse de 1 mm/s, 2 mm/s et 3 mm/s. Dans tous les cas, les deux transducteurs étaient pilotés par des impulsions de 8 cycles à 2,5 kW, engendrant une pression négative de pic de -15 MPa. Le premier protocole s'est avéré optimal, conduisant à une recanalisation efficace (80±7% du débit maximal) après 3 passages, pour une durée de traitement de 90s. Un très faible nombre de débris de taille supérieure à 100 µm (1,6±1,7 per thrombus) a été détecté, mais aucun de taille supérieure à 200 µm, ce qui peut être considéré sans danger d'embolie.

Un transducteur ultrasonore d'imagerie TUl, par exemple une sonde sonographique bi- ou tridimensionnelle, est agencé au centre du transducteur de thérapie, à l'intérieur de l'élément de plus petit rayon E4. Ce transducteur présente un champ de vision CV qui s'étend principalement selon l'axe z et inclut la région où se trouve normalement la tache focale TFU. Dans le mode de réalisation de la figure 1, plus précisément, le transducteur ultrasonore d'imagerie TUl est du type bidimensionnel et le champ de vision se trouve dans un plan xz. Le système d'imagerie de contrôle SEC pilote le transducteur d'imagerie, reçoit les signaux Im acquis par ce dernier et les traite pour reconstruire des images d'une région à traiter RAT du corps C.

Avantageusement, le transducteur d'imagerie dépasse axialement (c'est-à-dire suivant la direction z) du bord extérieur du transducteur de thérapie. Par exemple, il peut dépasser de 10 - 50 mm et préférentiellement de 10 - 25 mm. L'interface acoustique lA peut présenter une ouverture lui permettant d'être traversée par le transducteur d'imagerie ou, comme dans la cas de la figure 1, un écrasement en correspondance de ce dernier. Cela permet à une face active du transducteur d'imagerie d'être en contact « direct » (en fait, par l'intermédiaire d'une fine couche de gel de couplage acoustique) ou « indirect » (à travers une double épaisseur de la poche d'interface acoustique lA, et également de la fine couche de gel) avec la surface du SC du corps C, alors que le bord du transducteur de thérapie peut être espacé de cette surface.

Dans le mode de réalisation de la figure 1, le transducteur de thérapie TUT et le transducteur d'imagerie TUI sont reliés par l'intermédiaire d'un système mécanique SMDR permettent leur déplacement relatif en translation suivant la direction z. Par exemple, le transducteur d'imagerie peut être fixé à une base de ce système mécanique, et le transducteur de thérapie être relié à cette base par l'intermédiaire de trois vérins (deux seulement sont visibles sur la figure) orientés selon l'axe z. Le pilotage synchrone des trois vérins par le système électronique de contrôle SEC permet de déplacer le transducteur de thérapie - et donc la tache focale TFU des ultrasons focalisés - selon la direction z, sans qu'il ne soit nécessaire de déplacer en même temps le transducteur d'imagerie. Le système mécanique de déplacement relatif SMDR également peut prendre d'autres formes, par exemple il peut se baser sur un système pignon-crémaillère.

La présence du système mécanique SMDR n'est pas essentielle : en effet, la structure multiéléments du transducteur de thérapie et le circuit formateur de faisceau FEF permettent également de déplacer la tache focale TFU des ultrasons focalisés selon la direction z relativement au transducteur d'imagerie. Réciproquement, le système mécanique SMDR peut suffire, auquel cas il n'est pas nécessaire de prévoir un transducteur multiéléments et un système de focalisation électronique. Dans tous les cas, dans un appareil selon l'invention il est possible de positionner la tache focale TFU dans la direction axiale sans avoir à déplacer le transducteur d'imagerie. Cela n'est en revanche pas possible dans les appareils décrit dans la demande internationale WO 2009/094554 et dans les articles précités de Xi Zhang et al., où le positionnement de la tache focale ne peut être obtenu qu'en déplaçant solidairement le transducteur de thérapie et le transducteur d'imagerie.

Dans le mode de réalisation de la figure 1, le transducteur d'imagerie TUl est relié à la base du système mécanique SMDR - ou plus généralement à une pièce de support - par l'intermédiaire d'un capteur de force CF, par exemple piézoélectrique, permettant de mesurer une force s'exerçant sur le transducteur dans la direction z. Le signal de mesure de force SF acquis par le capteur est transmis au système électronique de contrôle SEC. Cela a une double fonction : d'une part, la détection d'une force non nulle permet d'assurer que le transducteur d'imagerie est bien en contact - direct ou indirect - avec la surface SC du corps C ; d'autre part, la mesure quantitative de cette force permet d'éviter que le transducteur d'imagerie n'appuie trop fort sur la surface SC, écrasant les tissus du corps C.

La présence du capteur de force CF n'est pas essentielle. En effet il est également possible d'utiliser des techniques d'analyse des images reconstituées par le système électronique de contrôle pour détecter a surface SC et en déterminer la position par rapport au transducteur TUl.

L'ensemble comprenant le transducteur de thérapie TUT, le transducteur d'imagerie TUI et, le cas échéant, le système mécanique de déplacement relatif SMDR et/ou le capteur de force CF, est fixé à un système mécanique motorisé SMM, piloté par le système électronique de contrôle SEC, permettant son déplacement par rapport à un bâti (et donc par rapport au corps C) selon au moins deux degrés de liberté :
- une translation selon un premier axe AD1, parallèle à l'axe z ; et
- une translation ou une rotation selon ou autour un deuxième axe AD2, non parallèle - et typiquement perpendiculaire - au premier.

Dans le mode de réalisation de la figure 1 le deuxième degré de liberté est une translation suivant un deuxième axe AD2 parallèle à la direction x. Plus précisément, dans ce mode de réalisation, le système mécanique motorisé SMM comprend un chariot coulissant le long d'un rail orienté suivant l'axe AD2 (x) et portant un vérin assurant la translation selon l'axe AD1 (z). Dans des modes de réalisation plus sophistiqué plusieurs degrés de liberté peuvent être prévus - par exemple trois translations et trois rotations, et plus particulièrement trois translations suivant les axes orthogonaux x, y et z et trois rotations autour de ces mêmes axes. Cela peut être obtenu, entre autres, au moyen d'un bras robotique.

Comme cela a été exposé plus haut, le système électronique de contrôle SEC assure une pluralité de fonctions : pilotage du système mécanique de déplacement relatif SMDR et du système mécanique motorisé SMM, pilotage des transducteurs de thérapie et d'imagerie, acquisition des signaux d'imagerie, reconstruction et analyse des images, acquisition des signaux de mesure de force... Ce système peut comprendre un ou plusieurs ordinateurs et/ou cartes électroniques numériques dédiées. Ces éléments ne doivent pas nécessairement être co-localisés, mais peuvent notamment être reliés par l'intermédiaire d'un bus, un réseau local ou même l'Internet.

Un appareil selon l'invention peut être utilisé pour mettre en oeuvre un procédé de traitement d'une thrombose conformément à la figure 2.

Premièrement - étape a) - le système mécanique motorisé SMM est piloté par le système électronique de contrôle SEC pour effectuer un premier balayage de la surface SC du corps C en correspondance de la région à traiter RAT. Simultanément - étape b) - le transducteur ultrasonore d'imagerie TUI est utilisé pour acquérir une pluralité d'images de la région RAT, de préférence permettant une reconstruction tridimensionnelle de cette dernière. Le pilotage du système mécanique motorisé SMM est effectué de telle sorte que le transducteur d'imagerie soit maintenu à une distance constante de la surface SC, avec une tolérance prédéfinie qui est généralement inférieure ou égale à 10% et de préférence à 1%. Cette distance est typiquement comprise entre 0 (contact direct ou indirect, ce qui constitue le mode de réalisation préféré) et 40 mm, de manière à assurer une qualité d'image satisfaisante et constante au cours du balayage. Comme évoqué plus haut, en outre, lorsque le transducteur d'imagerie est maintenu en contact avec la surface SC, un signal de mesure de force SF peut être utilisé pour maintenir constante (également avec une tolérance généralement inférieure ou égale à 10% et de préférence à 1%) la pression exercée par le transducteur sur ladite surface.

Pendant ce premier balayage, le transducteur de thérapie est maintenu inactif.

Ensuite - étape b1) les images acquises au cours du premier balayage sont analysées par le système électronique de contrôle SEC afin d'identifier automatiquement un vaisseau sanguin à traiter (référence V sur la figure 1) dans la région RAT. Cette étape peut être mise en oeuvre en choisissant ou en reconstituant une vue en coupe selon un plan (plan yz dans le cas de la figure 1) approximativement perpendiculaire à l'axe du vaisseau à détecter, de telle sorte que le vaisseau apparaisse comme un cercle ou une ellipse, et en appliquant un algorithme de détection de cercles, basé par exemple sur une transformée de Hough ou une détection de contours de type Canny-Dériche associée à un accumulateur qui détermine la position du centre du cercle par suffrage. D'autres algorithmes connus peuvent également être utilisés. En variante, la détection du vaisseau sanguin peut être effectuée par un opérateur, le cas échéant à l'aide d'un seuillage des images acquises et/ou d'une détection de contour. Puis - étape b2) - un chemin ou trajectoire à l'intérieur du corps C et suivant le vaisseau sanguin V identifié est déterminé. Cette opération peut être effectuée automatiquement par le système électronique de contrôle SEC, ou manuellement par un opérateur.

Une fois terminées ces opérations préparatoires, le traitement thérapeutique proprement dit peut commencer.

Le système mécanique motorisé SMM est piloté à nouveau par le système électronique de contrôle SEC pour effectuer un deuxième balayage de la surface SC du corps C - étape c). Comme dans le premier balayage, le pilotage s'effectue de telle sorte que le transducteur d'imagerie soit maintenu à une distance constante de la surface SC (préférentiellement comprise entre 0 - cas préféré - et 40 mm avec une tolérance non supérieure à 10% voire à 1%). Egalement comme dans le premier balayage, lorsque le transducteur d'imagerie est maintenu en contact avec la surface SC, un signal de mesure de force SF peut être utilisé pour maintenir constante (également avec une tolérance généralement inférieure ou égale à 10% et de préférence à 1%) la pression exercée par le transducteur sur ladite surface.

Le deuxième balayage n'est généralement pas identique au premier. En effet, alors que le premier balayage vise à permettre une exploration exhaustive de la région à traiter contenant le vaisseau V à traiter, lors du deuxième balayage le système mécanique motorisé SMM est piloté de telle sorte que le vaisseau sanguin V se trouve dans la région centrale (par exemple, dans le tiers central selon l'axe z et selon un axe perpendiculaire à z) du champ de vision CV du transducteur d'imagerie. Avantageusement, des images acquises au cours du deuxième balayage sont utilisées pour effectuer le pilotage du système mécanique motorisé en boucle fermée.

Pendant le deuxième balayage, le transducteur ultrasonore de thérapie TUT est activé et le système mécanique de déplacement relatif SMDR et/ou le circuit de génération de faisceau FEF sont pilotés de telle sorte que la tache focale TFU des ondes ultrasonores focalisées émises par le transducteur TUT suive la trajectoire définie lors de l'étape b2). Cela constitue la dernière étape - d) - du procédé de la figure 2.

Par ailleurs, au cours ou après le deuxième balayage, le transducteur ultrasonore d'imagerie peut être utilisé, sous le contrôle du système électronique SEC, pour acquérir des images d'échographie Doppler afin de surveiller la recanalisation d'un vaisseau sanguin traité par imagerie Doppler.

## Revendications

1. Appareil de traitement de la thrombose vasculaire par ultrasons, comprenant :
- un transducteur ultrasonore de thérapie (TUT), adapté pour générer des ondes ultrasonores focalisées (OUF) se propageant selon un axe d'émission (AE) ;
- un transducteur ultrasonore d'imagerie (TUI) associé au transducteur ultrasonore de thérapie, adapté pour acquérir des images (Im) bi- ou tridimensionnelles d'une région à traiter (RAT) d'un corps (C) humain ou animal, la région à traiter incluant une tache focale (TFU) du transducteur ultrasonore de thérapie ;
**caractérisé en ce qu'**il comprend également:
- un moyen de déplacement (SMDR, FEF) de la tache focale du transducteur ultrasonore de thérapie suivant l'axe d'émission par rapport au transducteur ultrasonore d'imagerie ;
- un système mécanique motorisé (SMM) adapté pour déplacer le transducteur ultrasonore de thérapie et le transducteur ultrasonore d'imagerie en translation suivant au moins un premier axe de déplacement (AD1) parallèle audit axe d'émission, et en translation ou en rotation selon ou autour un deuxième axe de déplacement (AD2) non parallèle au premier ; et
- un système électronique de contrôle (SEC) configuré pour :
- piloter le système mécanique motorisé de manière à effectuer un balayage de la région à traiter tout en maintenant constante, avec une tolérance prédéfinie, une distance entre le transducteur ultrasonore d'imagerie et une surface (SC) du corps humain ou animal ; et
- piloter le moyen de déplacement la tache focale du transducteur ultrasonore de thérapie de manière à contrôler la position de la tâche focale selon l'axe d'émission au cours dudit balayage.

2. Appareil selon la revendication 1 comprenant également un capteur de force (CF) adapté pour générer un signal (Sf) indicatif d'une force s'exerçant sur le transducteur ultrasonore d'imagerie suivant une direction parallèle audit axe d'émission, le système électronique de contrôle étant configuré pour acquérir ce signal et l'utiliser pour piloter le système mécanique motorisé de manière à maintenir ledit transducteur ultrasonore d'imagerie en contact avec ladite surface du corps humain ou animal au cours du balayage.

3. Appareil selon la revendication 2, dans lequel le système électronique de contrôle est également configuré pour appliquer ledit transducteur ultrasonore d'imagerie sur ladite surface du corps humain ou animal au cours du balayage avec une force constante.

4. Appareil selon l'une des revendications précédentes dans lequel le système électronique de contrôle est également configuré pour analyser des images acquises par le transducteur ultrasonore de thérapie de manière à détecter la surface dudit corps humain ou animal afin de piloter le système mécanique motorisé lors dudit balayage.

5. Appareil selon l'une des revendications précédentes dans lequel le système électronique de contrôle est également configuré pour piloter le moyen de déplacement de la tache focale du transducteur ultrasonore de thérapie de telle sorte que ladite tache focale suive un chemin (CTF) prédéfini à l'intérieur dudit corps humain ou animal au cours du balayage.

6. Appareil selon la revendication 5 dans lequel le système électronique de contrôle est également configuré pour :
a) piloter le système mécanique motorisé de manière à effectuer un premier balayage de la région à traiter ;
b) au cours de ce premier balayage, acquérir une pluralité d'images de ladite région tout en maintenant le transducteur ultrasonore de thérapie inactif ;
c) piloter le système mécanique motorisé de manière à effectuer un second balayage de la région à traiter ;
d) au cours de ce deuxième balayage, activer le transducteur ultrasonore de thérapie et piloter le moyen de déplacement sa tache focale de telle sorte que ladite tache focale suive un chemin prédéfini à l'intérieur dudit corps humain, identifié à partir des images acquises au cours du premier balayage.

7. Appareil selon la revendication 6 dans lequel le système électronique de contrôle est également configuré pour :
b1) analyser les images acquises au cours du premier balayage pour identifier un vaisseau sanguin (V) dans la région à traiter dudit corps humain ou animal ; et
b2) déterminer ledit chemin prédéfini à l'intérieur dudit corps humain de telle sorte qu'il correspond audit vaisseau sanguin.

8. Appareil selon la revendication 7 dans lequel le système électronique de contrôle est également configuré pour piloter le système mécanique motorisé de telle manière que, au cours du deuxième balayage, ledit vaisseau sanguin se trouve dans une partie centrale d'un champ de vision (CV) du transducteur ultrasonore d'imagerie.

9. Appareil selon la revendication 8 dans lequel le système électronique de contrôle est également configuré pour acquérir une pluralité d'images de la région à traiter au cours du deuxième balayage et pour utiliser ces images pour piloter le système mécanique motorisé.

10. Appareil selon l'une des revendications précédentes dans lequel le transducteur ultrasonore de thérapie est un transducteur concentrique multi-annulaire , le moyen de déplacement de sa tache focale comprend un formateur électronique de faisceau (FEF) configuré pour piloter les éléments (E1, E2, E3, E4) dudit transducteur avec des retards variables afin d'émettre les ondes ultrasonores focalisées avec une longueur focale réglable et le transducteur ultrasonore d'imagerie est agencé au centre du transducteur ultrasonore de thérapie et dépasse, dans une direction parallèle audit axe d'émission, du transducteur ultrasonore de thérapie

11. Appareil selon l'une des revendications précédentes dans lequel le moyen de déplacement de la tache focale du transducteur ultrasonore de thérapie comprend un système mécanique (SMDR) permettant un déplacement relatif, selon ledit axe d'émission, du transducteur ultrasonore de thérapie et du transducteur ultrasonore d'imagerie.

12. Appareil selon l'une des revendications précédentes comprenant également un dispositif d'interface acoustique (IA) adapté pour coupler les ondes ultrasonores focalisées générées par le transducteur ultrasonore de thérapie à ladite surface du corps humain ou animal.

13. Appareil selon l'une des revendications précédentes dans lequel ledit système mécanique motorisé présente trois degrés de liberté en translation et trois degrés de liberté en rotation.

14. Appareil selon l'une des revendications précédentes dans lequel ledit transducteur ultrasonore de thérapie est adapté pour générer des ondes ultrasonores focalisées à une fréquence supérieure ou égale à 2 MHz.

15. Appareil selon l'une des revendications précédentes dans lequel le système électronique de contrôle et le transducteur ultrasonore d'imagerie sont également configurés pour surveiller la recanalisation d'un vaisseau sanguin traité par imagerie Doppler.

## Patentansprüche

1. Gerät zur Behandlung von Gefäßthrombose durch Ultraschall, das Folgendes umfasst:
- einen Therapie-Ultraschallwandler (TUT), geeignet zum Erzeugen fokussierter Ultraschallwellen (OUF), die sich entlang einer Sendeachse (AE) ausbreiten;
- einen mit dem Therapie-Ultraschallwandler assoziierten Bildgebungs-Ultraschallwandler (TUI), geeignet zum Erfassen von zwei- oder dreidimensionalen Bildern (Im) einer zu behandelnden Region (RAT) eines menschlichen oder tierischen Körpers (C), wobei die zu behandelnde Region einen Brennpunkt (TFU) des Therapie-Ultraschallwandlers einschließt;
**dadurch gekennzeichnet, dass** es auch Folgendes umfasst:
- ein Mittel (SMDR, FEF) zum Bewegen des Brennpunkts des Therapie-Ultraschallwandlers entlang der Sendeachse in Bezug auf den Bildgebungs-Ultraschallwandler;
- ein motorisiertes mechanisches System (SMM), geeignet zum translatorischen Bewegen des Therapie-Ultraschallwandlers und des Bildgebungs-Ultraschallwandlers entlang mindestens einer ersten Bewegungsachse (AD1) parallel zur Sendeachse, und zum translatorischen oder rotatorischen Bewegen entlang oder um eine zweite Bewegungsachse (AD2), die nicht parallel zur ersten ist; und
- ein elektronisches Steuersystem (SEC), konfiguriert zum:
- Ansteuern des motorisierten mechanischen Systems zum Durchführen eines Scans der zu behandelnden Region unter Konstanthaltung eines Abstands zwischen dem Bildgebungs-Ultraschallwandler und einer Oberfläche (SC) des menschlichen oder tierischen Körpers mit einer vordefinierten Toleranz; und
- Ansteuern des Mittels zum Bewegen des Brennpunkts des Therapie-Ultraschallwandlers, so dass die Position des Brennpunkts entlang der Sendeachse während des Scannens überwacht wird.

2. Gerät nach Anspruch 1, das auch einen Kraftsensor (CF) umfasst, geeignet zum Erzeugen eines Signals (Sf), das eine auf den Bildgebungs-Ultraschallwandler entlang einer Richtung parallel zur Sendeachse wirkende Kraft anzeigt, wobei das elektronische Steuersystem zum Erfassen und Nutzen dieses Signals zum Ansteuern des motorisierten mechanischen Systems konfiguriert ist, so dass der Bildgebungs-Ultraschallwandler während des Scannens in Kontakt mit der Oberfläche des menschlichen oder tierischen Körpers gehalten wird.

3. Gerät nach Anspruch 2, wobei das elektronische Steuersystem auch zum Aufbringen des Bildgebungs-Ultraschallwandlers während des Scannens mit einer konstanten Kraft auf die Oberfläche des menschlichen oder tierischen Körpers konfiguriert ist.

4. Gerät nach einem der vorhergehenden Ansprüche, wobei das elektronische Steuersystem auch zum Analysieren der vom Therapie-Ultraschallwandler erfassten Bilder zum Erkennen der Oberfläche des menschlichen oder tierischen Körpers konfiguriert ist, um das motorisierte mechanische System während des Scannens anzusteuern.

5. Gerät nach einem der vorhergehenden Ansprüche, wobei das elektronische Steuersystem auch zum Ansteuern des Mittels zum Bewegen des Brennpunkts des Ultraschall-Therapiewandlers konfiguriert ist, so dass der Brennpunkt während des Scannens einem vordefinierten Pfad (CTF) innerhalb des menschlichen oder tierischen Körpers folgt.

6. Gerät nach Anspruch 5, wobei das elektronische Steuersystem auch konfiguriert ist zum:
a) Ansteuern des motorisierten mechanischen Systems, so dass ein erster Scan der zu behandelnden Region durchgeführt wird;
b) Erfassen, während dieses ersten Scans, einer Vielzahl von Bildern der Region, während der Therapie-Ultraschallwandler inaktiv gehalten wird;
c) Ansteuern des motorisierten mechanischen Systems, so dass ein zweiter Scan der zu behandelnden Region durchgeführt wird;
d) Aktivieren, während dieses zweiten Scans, des Therapie-Ultraschallwandlers und Ansteuern des Mittels zum Bewegen seines Brennpunkts, so dass der Brennpunkt einem vordefinierten Pfad innerhalb des menschlichen Körpers folgt, der anhand der während des ersten Scans erfassten Bilder identifiziert wird.

7. Gerät nach Anspruch 6, wobei das elektronische Steuersystem auch konfiguriert ist zum:
b1) Analysieren der während des ersten Scans erfassten Bilder zum Identifizieren eines Blutgefäßes (V) in der zu behandelnden Region des menschlichen oder tierischen Körpers; und
b2) Bestimmen des vordefinierten Pfads innerhalb des menschlichen Körpers, so dass er mit dem Blutgefäß übereinstimmt.

8. Gerät nach Anspruch 7, wobei das elektronische Steuersystem auch zum Ansteuern des motorisierten mechanischen Systems konfiguriert ist, so dass sich das Blutgefäß während des zweiten Scans in einem zentralen Teil eines Sichtfeldes (CV) des Bildgebungs-Ultraschallwandlers befindet.

9. Gerät nach Anspruch 8, wobei das elektronische Steuersystem auch zum Erfassen, während des zweiten Scans, einer Vielzahl von Bildern der zu behandelnden Region und zum Nutzen dieser Bilder zum Ansteuern des motorisierten mechanischen Systems konfiguriert ist.

10. Gerät nach einem der vorhergehenden Ansprüche, wobei der Therapie-Ultraschallwandler ein mehrringiger konzentrischer Wandler ist, das Mittel zum Bewegen seines Brennpunkts einen elektronischen Strahlformer (FEF) umfasst, der zum Ansteuern der Elemente (E1, E2, E3, E4) des Wandlers mit variablen Verzögerungen konfiguriert ist, um die fokussierten Ultraschallwellen mit einer regelbaren Brennweite auszusenden, und der Bildgebungs-Ultraschallwandler in der Mitte des therapeutischen Ultraschallwandlers angeordnet ist und in einer Richtung parallel zur Sendeachse aus dem therapeutischen Ultraschallwandler herausragt.

11. Gerät nach einem der vorhergehenden Ansprüche, wobei das Mittel zum Bewegen des Brennpunkts des Therapie-Ultraschallwandlers ein mechanisches System (SMDR) umfasst, das eine relative Bewegung des Therapie-Ultraschallwandlers und des Bildgebungs-Ultraschallwandlers entlang der Sendeachse zulässt.

12. Gerät nach einem der vorhergehenden Ansprüche, das auch eine akustische Schnittstellenvorrichtung (IA) umfasst, die zum Koppeln der vom Therapie-Ultraschallwandler erzeugten fokussierten Ultraschallwellen an die Oberfläche des menschlichen oder tierischen Körpers geeignet ist.

13. Gerät nach einem der vorhergehenden Ansprüche, wobei das motorisierte mechanische System drei translatorische Freiheitsgrade und drei rotatorische Freiheitsgrade aufweist.

14. Gerät nach einem der vorhergehenden Ansprüche, wobei der Therapie-Ultraschallwandler zum Erzeugen fokussierter Ultraschallwellen mit einer Frequenz von 2 MHz oder höher geeignet ist.

15. Gerät nach einem der vorhergehenden Ansprüche, wobei das elektronische Steuersystem und der Bildgebungs-Ultraschallwandler auch zum Überwachen der Rekanalisierung eines behandelten Blutgefäßes durch Doppler-Bildgebung konfiguriert sind.

## Claims

1. An apparatus for treating vascular thrombosis with ultrasound, comprising:
- a therapeutic ultrasonic transducer (TUT), suitable for generating focused ultrasonic waves (OUF) that propagate along an emission axis (AE);
- an imaging ultrasonic transducer (TUI) associated with the therapeutic ultrasonic transducer, suitable for acquiring two- or three-dimensional images (Im) of a region to be treated (RAT) of a human or animal body (C), the region to be treated including a focal spot (TFU) of the therapeutic ultrasonic transducer;
**characterised in that** in also comprises:
- a means (SMDR, FEF) for moving the focal spot of the therapeutic ultrasonic transducer along the emission axis with respect to the imaging ultrasonic transducer;
- a motorised mechanical system (SMM) suitable for moving the therapeutic ultrasonic transducer and the imaging ultrasonic transducer translationally along at least a first axis of movement (AD1) parallel to said emission axis, and translationally or rotationally along or about a second axis of movement (AD2) not parallel to the first; and
- an electronic control system (SEC) configured to:
- drive the motorised mechanical system so as to perform a scan of the region to be treated while keeping constant, with a predefined tolerance, a distance between the imaging ultrasonic transducer and a surface (SC) of the human or animal body; and
- drive the means for moving the focal spot of the therapeutic ultrasonic transducer so as to control the position of the focal spot along the emission axis during said scan.

2. The apparatus according to claim 1, also comprising a force sensor (CF) suitable for generating a signal (Sf) indicative of a force exerted on the imaging ultrasonic transducer along a direction parallel to said emission axis, the electronic control system being configured to acquire this signal and to use it to drive the motorised mechanical system so as to keep said imaging ultrasonic transducer in contact with said surface of the human or animal body during the scan.

3. The apparatus according to claim 2, wherein the electronic control system is also configured to apply said imaging ultrasonic transducer against said surface of the human or animal body during the scan with a constant force.

4. The apparatus according to one of the preceding claims, wherein the electronic control system is also configured to analyse images acquired by the therapeutic ultrasonic transducer so as to detect the surface of said human or animal body in order to drive the motorised mechanical system during said scan.

5. The apparatus according to one of the preceding claims, wherein the electronic control system is also configured to drive the means for moving the focal spot of the therapeutic ultrasonic transducer in such a way that said focal spot follows a predefined path (CTF) inside said human or animal body during the scan.

6. The apparatus according to claim 5, wherein the electronic control system is also configured to:
a) drive the motorised mechanical system so as to perform a first scan of the region to be treated;
b) during this first scan, acquire a plurality of images of said region while keeping the therapeutic ultrasonic transducer inactive;
c) drive the motorised mechanical system so as to perform a second scan of the region to be treated;
d) during this second scan, activate the therapeutic ultrasonic transducer and drive the means for moving its focal spot in such a way that said focal spot follows a predefined path inside said human body, which is identified from the images acquired during the first scan.

7. The apparatus according to claim 6, wherein the electronic control system is also configured to:
b1) analyse the images acquired during the first scan to identify a blood vessel (V) in the region to be treated of said human or animal body; and
b2) determine said predefined path inside said human body in such a way that it corresponds to said blood vessel.

8. The apparatus according to claim 7, wherein the electronic control system is also configured to drive the motorised mechanical system in such a way that, during the second scan, said blood vessel is located in a central portion of a field of view (CV) of the imaging ultrasonic transducer.

9. The apparatus according to claim 8, wherein the electronic control system is also configured to acquire a plurality of images of the region to be treated during the second scan and to use these images to drive the motorised mechanical system.

10. The apparatus according to one of the preceding claims, wherein the therapeutic ultrasonic transducer is a multi-ring annular transducer, the means for moving its focal spot comprises an electronic beam former (FEF) configured to drive the elements (E1, E2, E3, E4) of said transducer with variable delays in order to emit the focused ultrasonic waves with an adjustable focal length, and the imaging ultrasonic transducer is arranged at the centre of the therapeutic ultrasonic transducer and extends, in a direction parallel to said emission axis, beyond the therapeutic ultrasonic transducer.

11. The apparatus according to one of the preceding claims, wherein the means for moving the focal spot of the therapeutic ultrasonic transducer comprises a mechanical system (SMDR) allowing a relative movement, along said emission axis, of the therapeutic ultrasonic transducer and of the imaging ultrasonic transducer.

12. The apparatus according to one of the preceding claims, also comprising an acoustic interface device (IA) suitable for coupling the focused ultrasonic waves generated by the therapeutic ultrasonic transducer to said surface of the human or animal body.

13. The apparatus according to one of the preceding claims, wherein said motorised mechanical system has three degrees of freedom translationally and three degrees of freedom rotationally.

14. The apparatus according to one of the preceding claims, wherein said therapeutic ultrasonic transducer is suitable for generating focused ultrasonic waves at a frequency higher than or equal to 2 MHz.

15. The apparatus according to one of the preceding claims, wherein the electronic control system and the imaging ultrasonic transducer are also configured to monitor recanalisation of a treated blood vessel by Doppler imaging.
